# EUROPÄISCHE PATENTSCHRIFT

(11) **EP 2 444 037 B1**
(45) Veröffentlichungstag und Bekanntmachung des Hinweises auf die Patenterteilung: **12.12.2012**
(21) Anmeldenummer: 11007733.6
(22) Anmeldetag: 22.09.2011
(51) Int. Cl.: A61F 5/37

(54) **Dynamische Schultergelenksorthese, insbesondere Schulterabduktionsorthese**
Dynamic shoulder joint orthotic, in particular shoulder abduction orthotic
Orthèse d'articulation de l'épaule dynamique, notamment orthèse d'abduction d'épaule

(30) Priorität: 21.10.2010 DE 102010049189
(43) Veröffentlichungstag der Anmeldung: 25.04.2012
(73) Patentinhaber: Albrecht GmbH, 83115 Neubeuern (DE)
(72) Erfinder: Opahle, Hans-Georg, 83024 Rosenheim (DE); Albrecht, Erich, 83115 Neubeuern (DE)
(74) Vertreter: Bauer, Friedrich

(56) Entgegenhaltungen:
- DE-U1- 8 407 242
- US-A- 2 661 000

## Beschreibung

Die Erfindung betrifft eine dynamische Schultergelenksorthese, insbesondere Schulterabduktionsorthese, gemäß dem Oberbegriff des Anspruches 1.

Das Schultergelenk des erwachsenen Menschen ist in ganz besonderem Maße gefährdet, bei nicht ausreichender Bewegung durch Kapselschrumpfung - besonders im Bereich des Recessus axillaris - und Verkleben der Verschiebestrukturen an Beweglichkeit zu verlieren und zwar vor allem im Sinne der Abspreizfähigkeit (Abduktion). Ein messbarer Immobilisierungsschaden einer ansonsten gesunden Schulter kann sich bereits nach einer Ruhigstellungsdauer von ca. einer Woche einstellen. Besonders gefährdet sind dabei Patienten im höheren Lebensalter. In Folge eines operativen Eingriffes mit einer Läsion der Kapselbandgewebe und der Gleitstrukturen ist das Risiko, eine Kontraktur des Schultergelenkes zu entwickeln, ungleich höher. Eine ausreichende Bewegung ist für die Funktion des Schultergelenkes jedoch extrem wichtig, um Kontrakturen zu vermeiden.

Sie fördert die Erguss- und Ödemresorption und hilft durch Beschleunigung des Blutflusses Thrombosen zu vermeiden.

Bei einer relativ großen Anzahl von Patienten wird während oder nach einer operativen Behandlung einer Erkrankung oder einer Traumafolge der Schulter festgestellt, dass eine erhebliche Einschränkung der Stabilität der die Abduktion des Oberarmes bewirkenden Strukturen vorliegt. Dies ist zum Beispiel der Fall nach einer Refixation oder Rekonstruktion der Sehne des Musculus supraspinatus durch Naht, wieder Anheften am Tuberculum majus, Rekonstruktion durch einen Latissimus dorsi Transfer oder Refixation des Tuberculum majus oder bei Frakturen des proximalen Humerus, bei deren Versorgung mittels Platte, intermedullärem Nagel oder Endoprothese die Tubercula mit den anhängenden Sehnen der Rotatorenmanschette refixiert werden mussten.

In der Nachbehandlung ist es regelmäßig für eine relativ lange Zeit, beispielsweise sechs Wochen, erforderlich, die Rekonstruierten Strukturen vor einer erneuten Ruptur, bzw. Redislokation zu schützen. Der Arm wird daher üblicherweise mittels eines Abduktionskissens oder einer Abduktionsorthese in einer Abspreizstellung zwischen 30° bis 60° (abhängig vom intraoperativ festgestellten Spannungszustand der refixierten Sehnen) immobilisiert. Um die vorstehenden, durch nicht ausreichende Bewegung verursachten Probleme so gering wie möglich zu halten, erfolgt eine Übungsbehandlung durch passive Bewegungen der Schulter, zu deren Ausführung eine Hilfsperson erforderlich ist, beispielsweise ein Physiotherapeut oder entsprechend instruierte Angehörige. Die Nachbehandlung kann außerdem durch die Verwendung eines CPM-Stuhles (CPM: Continuous Passiv Motion) unterstützt werden. Hierbei wird der Arm auf eine Lagerungsschale eines speziellen Therapiestuhles gelegt und in einem definierbaren Umfang mittels Motorkraft passiv im Schultergelenk durchbewegt. Dieses Verfahren ist jedoch sehr kostenintensiv und aufwendig.

Das passive Durchbewegen eines Schultergelenkes mit Hilfe eines Therapeuten ist jedoch wegen der zeitlichen Begrenzung nicht ausreichend geeignet, die im täglichen Gebrauch spontan ausgeführten Bewegungen zu ersetzen und einen Immobilisierungsschaden zuverlässig zu verhindern.

Aus der DE 84 07 242 U1 ist weiterhin eine dynamische Schultergelenksorthese in der Form einer Schulterabduktionsorthese gemäß dem Oberbegriff des Anspruches 1 bekannt. Diese Orthese ermöglicht es, die Oberarmschiene gegen den Widerstand einer Feder in Adduktions- und Abduktionsrichtung über einen bestimmten Schwenkbereich zu bewegen. Hierzu weist die Schiene eine am Oberkörper befestigbare Führungseinrichtung mit einem Gehäuse auf, in dem das untere Ende einer Stützstange verschiebbar geführt ist. Die Feder ist als Druckfeder ausgebildet und wird vom unteren Ende der Stützstange zunehmend zusammengedrückt, wenn der Oberarm, ausgehend von einer beispielsweise horizontal abgespreizten Stellung, nach unten in Adduktionsrichtung bewegt wird.

Eine weitere Orthese gemäß des Oberbegriffes von Anspruch 1 ist aus der US 2,661,000 bekannt.

Mit Hilfe derartiger Orthesen ist es möglich, die Orthese durch Auswahl einer geeigneten Feder so einzustellen, dass sie den Oberarm in einer bestimmten Abduktionsstellung hält oder in diese Stellung zurück führt, ohne dass der Patient die entsprechenden Muskeln aktivieren müsste. Nachteilig ist jedoch, dass sich die Stützkraft der Orthese bei einer Schwenkbewegung des Oberarms ändert. Bei einer Bewegung in Adduktionsrichtung wird die Feder zunehmend zusammengedrückt, wodurch sich die Federkraft erhöht und eine immer weiter zunehmende Muskelkraft erforderlich ist, um den Oberarm nach unten in die maximal abgesenkte Stellung zu bewegen. Umgekehrt drückt die Feder die Oberarmschiene aus ihrer untersten Stellung zunächst mit sehr großer Federkraft in Abduktionsrichtung, während die Federkraft mit zunehmender Entspannung der Feder abnimmt. Die Federunterstützung variiert somit über den Federweg stark. Dies ist jedoch insbesondere in denjenigen Fällen, in denen es auf eine passive Mobilisierung des Schultergelenks und damit zusammenwirkender Sehnen und Muskeln ankommt, unerwünscht. Im Gegensatz zur Wirkung der bekannten Abduktionsorthese kann es auch häufig wünschenswert sein, die Stützkraft mit zunehmendem Abspreizwinkel des Oberarms sogar zu erhöhen, da dann die auf die Oberarmschiene drückende Gewichtskraft des Oberarms größer wird.

Der Erfindung liegt die Aufgabe zu Grunde, eine dynamische Schultergelenksorthese der eingangs genannten Art zu schaffen, mit der eine Mobilisierungsbehandlung der Schulter auf möglichst effektive und physiologisch optimale Weise möglich ist.

Diese Aufgabe wird erfindungsgemäß durch eine dynamische Schultergelenksorthese mit den Merkmalen des Anspruches 1 gelöst. Vorteilhafte Ausführungsformen der Erfindung sind in den weiteren Ansprüchen beschrieben.

Bei der erfindungsgemäßen Schultergelenksorthese ist zwischen der Feder und der Stützstange ein Ausgleichsmechanismus vorgesehen, welcher der Federkraftveränderung entgegenwirkt, die beim Schwenken der Oberarmschiene auftritt, so dass die zum Schwenken der Oberarmschiene in Adduktionsrichtung und/oder Abduktionsrichtung aufzubringende Kraft über den gesamten Schwenkbereich zumindest annähernd gleich bleibt.

Die erfindungsgemäße Schultergelenksorthese ermöglicht eine sehr effektive Mobilisierungsbehandlung des Schultergelenks. Wird das Gelenk in dem vom Operateur als günstig eingestuften Bewegungsumfang freigegeben und die Federkraft so eingestellt, dass die Eigenschwere des darauf gelagerten Armes gerade überwunden wird, kann durch den Patienten insbesondere eine eigenständige, für die Adduktoren belastungsfreie Mobilisierung des Schultergelenkes ausgeführt werden. Insbesondere ist es möglich, dass durch eine bereits geringe Aktivierung der in der Regel nicht beeinträchtigen Adduktoren der Schulter (M. Latissimus dorsi, M. Pectoralis major, M. Subscapularis, M. Teres major und minor) der Arm im Schultergelenk bis zum definierten Ausgangspunkt (beispielsweise maximal 30°) adduziert und nach Entspannen der Adduktoren über die Federkraft bis zum definierten Endpunkt (in der Regel 90°) abduziert wird.

Hierbei wird das "Kleinert-Prinzip" verwirklicht, d. h. bei der Aktivierung der Agonisten werden reflektorisch die Antagonisten entspannt. In diesem Fall werden bei Aktivierung der Adduktoren die Abduktoren entspannt und die Abduktion wird anschließend durch die Federkraft ausgeführt, so dass die schutzbedürftigen Strukturen bei der Bewegung des Schultergelenkes stets ohne Zugbelastung bleiben und nicht in ihrer Einheilung gestört werden.

Von besonderem Vorteil ist, dass diese passive, für die Abduktoren der Schulter belastungsfreie Mobilisierung von Patienten jederzeit ohne weitere technische oder personelle Hilfen ausgeführt werden kann.

Liegt bereits eine Abspreizhemmung des Schultergelenkes (Abduktionskontraktur) durch eine Verklebung und Vernarbung des Recessus axilaris und der Sehnen vor, ist es möglich, durch eine entsprechende Einstellung der Federkraft deutlich über die Eigenschwere des Armes eine forcierte Abduktion zu bewirken. Hierdurch ist im Sinne einer Quengelungsbehandlung ein sukzessives Aufdehnen der Schulter und damit eine Steigerung des Bewegungsumfanges des Gelenkes erreichbar.

Gemäß einer vorteilhaften Ausführungsform umfasst der Ausgleichsmechanismus eine um eine Drehachse drehbare Kurvenscheibe, die mit einem mit dem unteren Ende der Stützstange in Wirkverbindung stehenden Kraftübertragungselement, vorzugsweise einem Seilzug, bewegungsgekoppelt ist, wobei zwischen Kraftübertragungselement und Drehachse der Kurvenscheibe ein Hebel wirkt, dessen Länge mit zunehmender Federkraft größer wird. Bei dieser Ausführungsform verändert sich somit in Abhängigkeit der Drehstellung der Kurvenscheibe die Länge des wirksamen Hebelarms, über den vom Kraftübertragungselement ein Drehmoment auf die Kurvenscheibe ausgeübt wird. Soll beispielsweise von der Feder eine konstante Kraft auf das untere Ende der Stützstange über den gesamten Verschiebeweg ausgeübt werden, kann die wirksame Hebellänge, über welche das Kraftübertragungselement auf die Kurvenscheibe ein Drehmoment ausübt, mit zunehmenden Drehwinkel der Kurvenscheibe und damit einhergehend mit zunehmender Federkraft zunehmend vergrößert werden, wodurch das vom Kraftübertragungselement auf die Kurvenscheibe ausgeübte Drehmoment in gleicher Weise vergrößert wird wie dasjenige Drehmoment, welches die Feder auf die Kurvenscheibe ausübt.

Selbstverständlich ist es auch möglich, den Ausgleichsmechanismus so einzustellen, dass die von der Feder auf das untere Ende der Stützstange übertragene Verschiebekraft nicht über den gesamten Verschiebeweg gleich bleibt, sondern sich gegebenenfalls an die momentan auf die Oberarmschiene wirkende, sich je nach Winkelstellung ändernde Gewichtskraft des Oberarms anpasst. Insbesondere ist es möglich, die von der Feder auf das untere Ende der Stützstange übertragene Verschiebekraft so einzustellen, dass sie an jeder Stelle des Verschiebewegs derjenigen Kraft entspricht, die notwendig ist, um in jeder Adduktions- bzw. Abduktionsstellung des Oberarms die Eigenschwere des Armes zu überwinden und so eine Bewegung des Arms ohne nennenswerte Beanspruchung der Muskeln zu ermöglichen.

Gemäß einer vorteilhaften Ausführungsform umfasst das Kraftübertragungselement einen Seilzug, der über einen Umfangsflächenabschnitt der Kurvenscheibe geführt ist, wobei sich der Abstand des Umfangsflächenabschnitts zur Drehachse der Kurvenscheibe längs des Umfangs der Kurvenscheibe ändert. Dies ermöglicht eine einfache, platzsparende und sehr zuverlässig funktionierende Bauweise der Orthese.

Vorteilhafterweise besteht die Feder aus einer Spiralfeder, deren Hauptebene parallel zur Hauptebene der Kurvenscheibe angeordnet ist. Hierdurch lässt sich ebenfalls eine sehr platzsparende und einfache Bauweise verwirklichen.

Gemäß einer vorteilhaften Ausführungsform ist die Spiralfeder an einem Ende drehfest mit einem Zahnrad gekoppelt, das zur Einstellung der Vorspannkraft der Spiralfeder im Gehäuse der Führungseinrichtung drehbar gelagert ist. Auf diese Weise kann die Vorspannkraft der Feder auf sehr einfache Weise verändert und an die jeweiligen Bedürfnisse angepasst werden, ohne die Feder wechseln zu müssen.

Vorteilhafterweise ist das Zahnrad mittels eines Schneckenrads drehbar, das über einen manuell betätigbaren Drehmechanismus drehbar ist. Hierdurch kann ein selbsthemmendes Getriebe zwischen Schneckenrad und Zahnrad geschaffen werden, das ohne weitere Blockierelemente die eingestellte Lage des Zahnrads aufrecht erhält.

Eine sehr präzise arbeitende Orthese und genaue Führung der Stützstange ergibt sich, wenn im Gehäuse der Führungseinrichtung ein Schlitten längs verschiebbar geführt ist, an dem das untere Ende der Stützstange angelenkt und an dem der Seilzug befestigt ist, so dass der Schlitten bei einer Abduktions- oder Adduktionsbewegung der Oberarmschiene sowohl mit der Oberarmschiene als auch mit der federbelasteten Kurvenscheibe bewegungsgekoppelt ist.

Vorteilhafterweise wird der Schwenkbereich der Oberarmschiene durch einen Schlittenwegbegrenzungsmechanismus begrenzt, der in oder am Gehäuse angeordnete, in Längsrichtung des Schlittenwegs verstellbare Anschläge aufweist. Zweckmäßigerweise umfasst hierzu der Schlittenwegbegrenzungsmechanismus zwei parallel im Gehäuse angeordnete Spindeln, die jeweils einen Anschlag in der Form einer Spindelmutter tragen. Hierdurch kann auf einfache Weise durch Drehen einer entsprechenden Spindel der zugeordnete Anschlag verstellt werden, um den Schwenkbereich der Oberarmschiene entsprechend zu verändern.

Die Erfindung wird nachfolgend anhand der Zeichnungen beispielhaft näher erläutert. Es zeigen:
- Figur 1:: eine Schultergelenksorthese gemäß der Erfindung in räumlicher Darstellung,
- Figur 2:: eine Explosionsdarstellung der Führungseinrichtung, Stützstange, Gelenkplatte und des Stützstangen-Kupplungsteils,
- Figur 3:: eine Vorderansicht des Kupplungsteils von Figur 2,
- Figur 4:: wesentliche Einzelteile der Führungseinrichtung,
- Figur 5:: die Einzelteile von Figur 4 im montierten Zustand,
- Figur 6:: einen Längsschnitt durch die Führungseinrichtung von Figur 5,
- Figur 7:: eine Seitenansicht der Führungseinrichtung, wobei sich Stützstange und Gelenkplatte in einer ersten, angehobenen Stellung befinden,
- Figur 8:: eine Darstellung gemäß Figur 7, wobei sich Stützstange und Gelenkplatte in einer zweiten, abgesenkten Stellung befinden,
- Figur 9:: die Kurvenscheibe und den Seilzug in einer Drehstellung der Kurvenscheibe, die sie bei einer Stellung der Orthese gemäß Figur 7 einnimmt,
- Figur 10:: die Kurvenscheibe und den Seilzug, wenn sich die Orthese in der Stellung von Figur 8 befindet,
- Figur 11:: eine Vorderansicht auf das Zahnrad der Führungseinrichtung, und
- Figur 12:: eine Seitenansicht auf das Zahnrad von Figur 11.

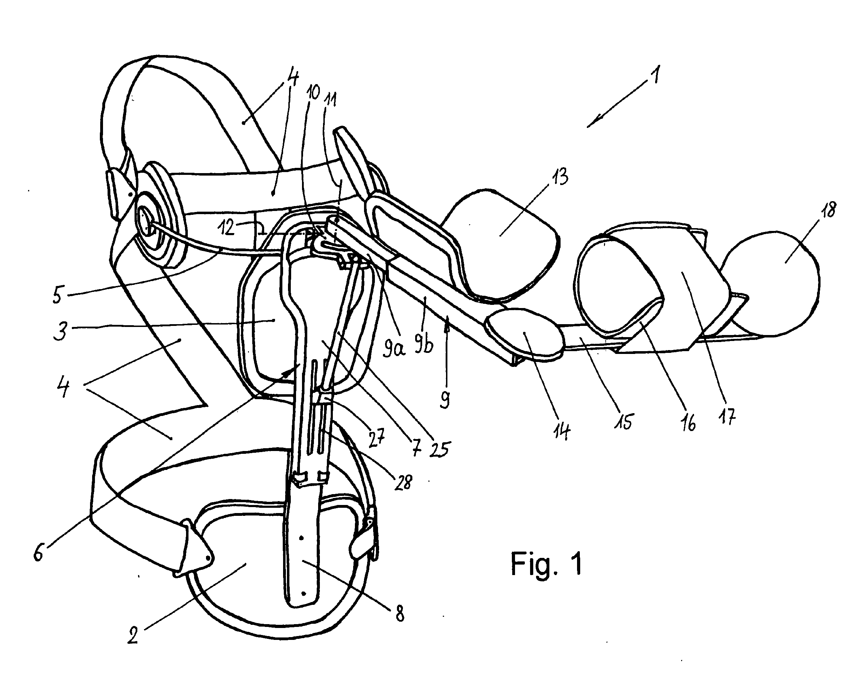

Die in Figur 1 dargestellte Schultergelenksorthese 1 umfasst ein unteres Abstützelement 2, das seitlich am Oberkörper im Hüftbereich angelegt wird, sowie ein oberes Anlageelement 3, das direkt unterhalb der Schulter seitlich am Brustkorb angelegt wird. Abstützelement 2 und Anlageelement 3 werden mittels eines Geschirrs am Oberkörper befestigt, das beim dargestellten Ausführungsbeispiel Gurte 4 und eine stabile Stahlspange 5 umfasst.

Abstützelement 2 und Anlageelement 3 dienen zur Befestigung einer stabilen Führungseinrichtung 6, die ein längliches Gehäuse 7 und eine daran befestigte Verlängerungsschiene 8 umfasst. Zweckmäßigerweise bestehen Gehäuse 7 und Verlängerungsschiene 8 aus Metall, beispielsweise Aluminium. Im angelegten Zustand der Schultergelenksorthese 1 verläuft die Längsachse des Gehäuses 7 und der Verlängerungsschiene 8 im Wesentlichen vertikal, wobei das Gehäuse 7 am oberen Anlageelement 3 befestigt ist und sich bis in die Nähe der Schulter, d.h. bis nahe zum Achselbereich des Patienten, erstreckt, während die Längsschiene 8 einerseits am unteren Ende des Gehäuses 7 und andererseits am unteren Abschnittselement 2 befestigt ist und je nach Länge den Abstand zwischen dem hüftnahen Abstützelement 2 und dem oberen Anlageelement 3 bestimmt.

Um den Arm eines Patienten in abgespreizter Stellung zu halten und/oder in Abduktions- und Adduktionsrichtung unterstützend zu führen, ist eine Oberarmschiene 9 mittels einer Gelenkplatte 10 gelenkig im oberen Endbereich des Gehäuses 7 gelagert. Zur Längenverstellung der Oberarmschiene 9 besteht diese aus zwei Schienenteilen 9a, 9b, die teleskopartig ineinander geführt sind und relativ zueinander verschoben werden können, um die Ausziehlänge zu variieren. Die Oberarmschiene 9 ist dabei endseitig relativ zur Gelenkplatte 10 auch um eine Schwenkachse 11 schwenkbar, so dass die Oberarmschiene 9 in unterschiedlichen Winkeln an der Gelenkplatte 10 fixiert werden kann. Hierzu dient ein in der Gelenkplatte 10 vorgesehenes kreisbogenförmiges Langloch 23, durch das eine nicht dargestellte Schraube hindurchgeführt werden kann, mit der das proximale Schienenteil 9a an der Gelenkplatte 10 festgelegt wird. Die Schwenkachse 11 verläuft senkrecht zur Schwenkachse 12, um welche die Gelenkplatte 10 relativ zum Gehäuse 7 in Abduktions- und Adduktionsrichtung verschwenkt werden kann.

An der Oberseite des Schienenteils 9b ist eine halbschalenförmige Oberarmauflage 13 befestigt, in die der Oberarm eingelegt werden kann. Bei einem Verschieben des distalen Schienenteils 9b relativ zum proximalen Schienenteil 9a bewegt sich somit die Oberarmauflage 13 entsprechend mit, so dass der Abstand zwischen Oberarmauflage 13 und dem Orthesengelenk verändert werden kann.

Die Oberarmschiene 9 ist an ihrem distalen Ende über ein unter einem Kissen 14 angeordneten Gelenk mit einer Unterarmschiene 15 verbunden. Auf dieser ist eine halbschalenförmige Unterarmauflage 16 befestigt, in die der Unterarm eingelegt werden kann. Mittels eines Befestigungsbandes 17 kann der Unterarm in der Unterarmauflage 16 fixiert werden. Am distalen Ende der Unterarmschiene 15 befindet sich eine Handauflage 18, die insbesondere die Form eines balligen oder kugeligen Kissens haben kann, das es dem Patienten ermöglicht, mit seinen Fingern Knetübungen durchzuführen.

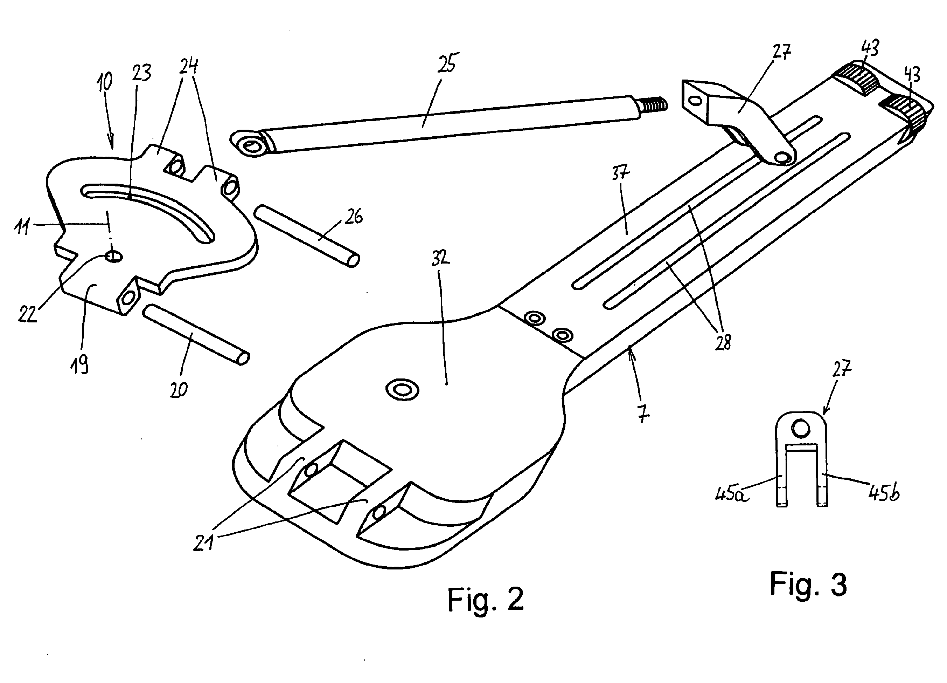

Die Gelenkplatte 10 bildet ein Scharniergelenk mit der Führungseinrichtung 6 und weist hierzu an einem Ende eine Gelenklasche 19 auf (Figur 2), die über einen Gelenkstift 20 gelenkig mit Lagerstegen 21 des Gehäuses 7 verbunden ist. Zur schwenkbaren Lagerung der Oberarmschiene 9 relativ zur Gelenkplatte 10 um die Schwenkachse 11 ist in der Nähe der Gelenklasche 19 eine Bohrung 22 vorgesehen, in die ein nicht dargestellter Gelenkbolzen eingeführt ist.

Die Oberarmschiene 9 ist im proximalen Endbereich des Schienenteils 9a an der Gelenkplatte 10 fixiert. Ein entsprechendes Verschwenken der Gelenkplatte 10 in Adduktions- und Abduktionsrichtung ist somit mit einem entsprechenden Verschwenken der Oberarmschiene 9 und damit auch der Unterarmschiene 15 gekoppelt.

Die Gelenkplatte 10 weist weiterhin an ihrem der Gelenklasche 19 gegenüberliegenden Ende zwei Gelenklaschen 24 auf, die zum gelenkigen Verbinden des oberen Endes einer Stützstange 25 dienen. Die Stützstange 25 ist hierzu mit dem oberen Ende zwischen die Gelenklaschen 24 eingeführt und mittels eines Gelenkstifts 26 festgelegt.

Die Oberarmschiene 9 und damit der darauf aufliegende Arm des Patienten wird mittels der Stützstange 25 in gewünschten Abspreizstellungen relativ zum Oberkörper gehalten oder übt bei entsprechenden Abduktions- und Adduktionsbewegungen auf die Gelenkplatte 10 und damit auf die Oberarmschiene 9 von unten her eine Stützkraft aus. Das untere Ende der Stützstange 25 greift über ein gabelförmiges Kupplungselement 25 in zwei parallele Längsschlitze 28 des Gehäuses 7 ein und kann gegen die Wirkung oder mit Unterstützung eines nachfolgend noch näher beschriebenen Federmechanismus entlang der Längsschlitze 28 verschoben werden. Die Federkraft kann dabei so eingestellt werden, dass die auf die Oberarmschiene 9 wirkende Stützkraft das Gewicht des Arms des Patienten in jeder Abspreizstelllung des Arm gerade kompensiert, so dass der Arm schwerelos, d. h. ohne nennenswerte aktive Muskelunterstützung, in Abduktionsrichtung bewegt werden kann. Weiterhin ist es auch möglich, die Federkraft so einzustellen, dass die durch den Federmechanismus aufgebrachte, auf die Stützstange 25 einwirkende Schiebekraft größer als die Gewichtskraft des Arms einschließlich der Armschiene ist, so dass eine aktive Quengelungsbehandlung des Schultergelenks in Abduktionsrichtung ermöglicht wird. Ferner ist eine Fixierung der Stützstange 25 in jeder beliebigen Lage des Verschiebebereichs möglich, d.h. es ist eine stufenlose Fixierung der Oberarmschiene 9 und damit eine statische Fixierung des Arms in jeder Abspreizstellung möglich.

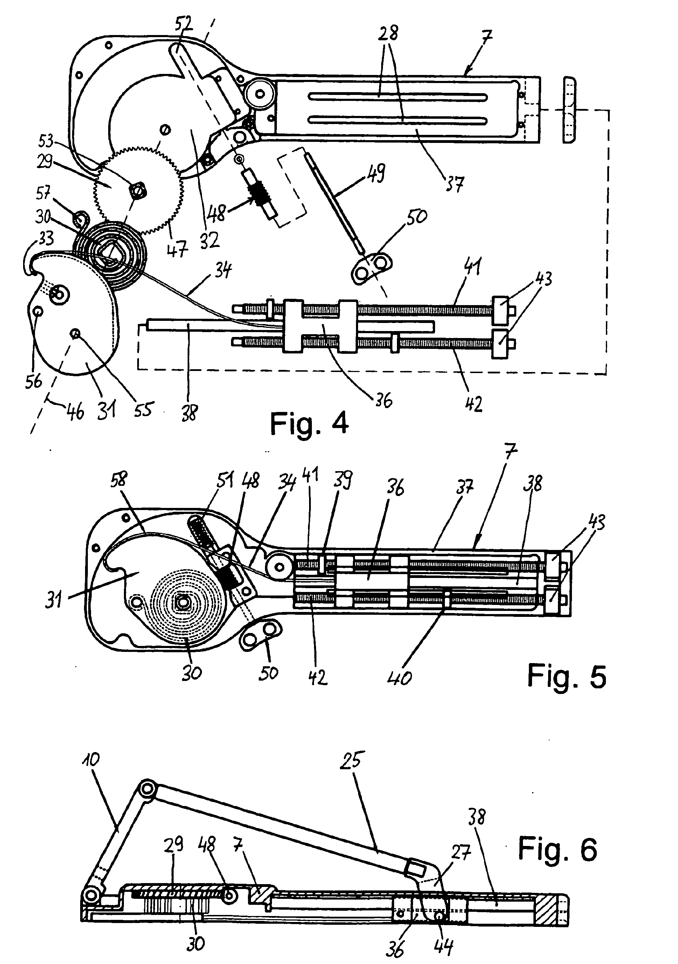

Im Folgenden wird anhand der Figuren 4 bis 6 der Aufbau und die Funktionsweise der Führungseinrichtung 6 näher beschrieben.

Die Führungseinrichtung 6 umfasst das Gehäuse 7 sowie ein Zahnrad 29, eine Feder 30 in der Form einer Spiralfeder und eine Kurven- oder Exzenterscheibe 31, die in einem Kopfteil 32 des Gehäuses 7 untergebracht sind. An der Kurvenscheibe 31 ist ein Ende 33 eines Seils 34 befestigt, dessen anderes Ende an einem Schlitten 36 befestigt ist.

Der Schlitten 36 ist in einem länglichen Gehäuseabschnitt 37 verschiebbar angeordnet, der sich an das Kopfteil 32 anschließt. Die Führung des Schlittens 36 erfolgt über einen mittig im Gehäuseabschnitt 37 stationär angeordneten Führungsstab 38, der durch eine entsprechende Längsbohrung im Schlitten 36 mit geringem Spiel hindurch geführt ist. Der Verschiebebereich des Schlitten 36 längs des Gehäuseabschnitts 37 wird durch Anschläge 39, 40 begrenzt, die als Spindelmuttern ausgebildet sind. Jeder Anschlag 39, 40 ist durch eine eigene Spindel 41, 42 in der Form von Gewindestäben verschiebbar, die parallel zueinander beidseits des Führungsstabs 38 im Gehäuseabschnitt 37 angeordnet sind. Da die Anschläge 39, 40 drehfest im Gehäuse 7 angeordnet sind, bewirkt ein Drehen der Spindeln 41, 42 ein Verschieben der Anschläge 39, 40 in Längsrichtung des Gehäuses 7. Das Drehen der Spindeln 41, 42 erfolgt über drehfest verbundene Rändelscheiben 43, die am Ende des Führungsabschnitt 37 über das Gehäuse 7 vorstehen (siehe auch Figur 2).

Im gezeigten Ausführungsbeispiel befindet sich der Anschlag 39 auf der dem Kopfteil 32 zugewandten Seite des Schlittens 36, während sich der Anschlag 40 auf der gegenüberliegenden Seite des Schlittens 36 befindet. Der Anschlag 39 begrenzt somit in Figur 5 den Verschiebeweg des Schlittens 30 nach links, d. h. in Figur 1 nach oben, und damit den Schwenkbereich der Oberarmschiene 9 in Abduktionsrichtung. Der Anschlag 40 begrenzt dagegen den Verschiebeweg des Schlittens 36 in Figur 5 nach recht, d.h. in Figur 1 nach unten, und damit den Schwenkbereich der Oberarmschiene 9 in Adduktionsrichtung.

Das Kupplungselement 27 ist über einen Querbolzen 44 (Figur 6) gelenkig mit dem Schlitten 36 verbunden. Hierbei durchdringen die beiden Seitenschenkel 45a, 45b (Figur 3) das Kupplungselement 27 die Längsschlitze 28 des Gehäuses 7. Eine Verschiebung des Schlittens 36 in Längsrichtung des Gehäuses 7 bewirkt somit eine entsprechende Verschiebung des unteren Endes der Stützstange 25.

Die Verschiebung des Schlittens 36 erfolgt gegen die bzw. mit Unterstützung der Federkraft der Feder 30, je nachdem, ob die Oberarmschiene 9 in Adduktionsrichtung oder in Abduktionsrichtung bewegt wird. Hierzu bringt die Feder 30 über die Kurvenscheibe 31 und das Seil 34 eine permanente Zugkraft auf den Schlitten 36 in Richtung Kopfteil 32 auf. Eine aus den Figuren 4, 5, 9, 10 ersichtliche, im Bereich zwischen der Kurvenscheibe 31 und dem Schlitten 36 drehbar im Gehäuse 7 gelagerte Seilführungsrolle 59 sorgt dafür, dass das Seil 34 parallel zur Verschieberichtung des Schlittens 36 in den Gehäuseabschnitt 37 eingeleitet wird.

Die Vorspannkraft der Feder 30 wird über das Zahnrad 29 eingestellt. Das Zahnrad 29 ist um eine querverlaufende Drehachse 46 (Figur 4) drehbar, die durch einen im Kopfteil 32 gelagerten, nicht dargestellten Querbolzen bestimmt wird. An seinem Umfang trägt das Zahnrad 29 eine Strinverzahnung 47, die mit einem Schneckenrad 48 zusammenwirkt. Das Schneckenrad 48 ist mit einer Welle 49 drehfest verbunden, welche das Schneckenrad 48 in Längsrichtung durchdringt. Die Welle 49 und damit das Schneckenrad 48 kann über ein blattförmiges Handhabungsteil 50 gedreht werden, das am außenliegenden Ende der Welle 49 angebracht ist. Durch Drehen des Schneckenrads 48 mittels der Welle 49 wird somit das Zahnrad 29 entsprechend um die Drehachse 46 gedreht. Aufgrund der selbsthemmenden Eigenschaft des Schneckengetriebes bleibt die eingestellte Drehstellung des Zahnrads 29 relativ zum Gehäuse 7 erhalten.

Aus Figur 5 ist weiterhin ersichtlich, dass am innenseitigen Ende der Welle 49 eine Druckfeder 51 vorgesehen ist, die in einer Ausnehmung 52 (Figur 4) des Gehäuses 7 aufgenommen ist. Das innenseitige Ende der Welle 49 steht in der in Figur 5 gezeigten Betätigungsstellung nur geringfügig in die Druckfeder 51 hinein und ist axial zur Druckfeder 51 hin abgestützt. Hierdurch ist es möglich, die Welle 49 zusammen mit dem Handhabungsteil 50 bei Nichtgebrauch axial entgegen der Druckkraft der Druckfeder 51 in das Gehäuse 7 hinein zu drücken, so dass das Handhabungsteil 50 nicht mehr über das Gehäuse 7 vorsteht.

Damit diese Verschiebung erfolgen kann, ist das unverschiebbar im Kopfteil 32 gelagerte Schneckenrad 48 axial verschiebbar, jedoch drehfest auf einem mittigen Mehrkantabschnitt, beispielsweise Vierkantabschnitt, der Welle 49 gelagert.

Die eingedrückte Stellung der Welle 49 wird mittels eines nicht dargestellten Rastmechanismus beibehalten. Zur Betätigung der Welle 49 wird der Rastmechanismus gelöst, wodurch die Druckfeder 51 die Welle 49 zusammen mit dem Handhabungsteil 50 in die in Figur 5 gezeigte Stellung verschiebt.

Wie aus den Figuren 4, 11, 12 ersichtlich, weist das Zahnrad 29 einen mittigen, axial vorspringenden Vierkantansatz 53 und einen daran anschließenden zylindrischen Ansatz 54 auf. Auf den Vierkantansatz 53 ist das innere, ebenfalls vierkantförmig gebogene Ende der Feder 30 aufgesteckt, das damit drehfest mit dem Zahnrad 29 verbunden ist. Auf dem zylinderförmigen Ansatz 54 des Zahnrads 29 ist die Kurvenscheibe 31 aufgesteckt, wobei der zylinderförmige Ansatz 54 eine Bohrung 55 der Kurvenscheibe 31 durchdringt. Die Kurvenscheibe 31 ist damit relativ zum Zahnrad 29 um die Drehachse 46 drehbar.

Die Kurvenscheibe 31 ist mittels eines Mitnahmebolzens 56, der in eine Öse 57 am äußeren Ende der Feder 30 eingreift, mit der Feder 30 gekoppelt. Durch die Vorspannkraft der Feder 30 wird damit ein Drehmoment auf die Kurvenscheibe 31 um die Drehachse 46 ausgeübt, das in dem in den Figuren 4 und 5 gezeigten Ausführungsbeispiel versucht, die Kurvenscheibe 31 im Gegenuhrzeigersinn zu drehen. Da das Seil 34 endseitig an der Kurvenscheibe 31 befestigt ist und längs des Umfangs, das heißt über einen Umfangsflächenabschnitt 58, der Kurvenscheibe 31 geführt ist, bewirkt dies einen Zug auf das Seil 34 und damit auf den Schlitten 36.

Es ist erkennbar, dass eine Abduktionsbewegung des Arms durch die Kraft unterstützt wird, mit der der Schlitten 36 mittels des Federmechanismus nach oben gezogen wird, während bei einer Adduktionsbewegung des Arms die Federkraft als Bremskraft wirkt, da der Schlitten 36 entgegen der Federkraft nach unten verschoben wird.

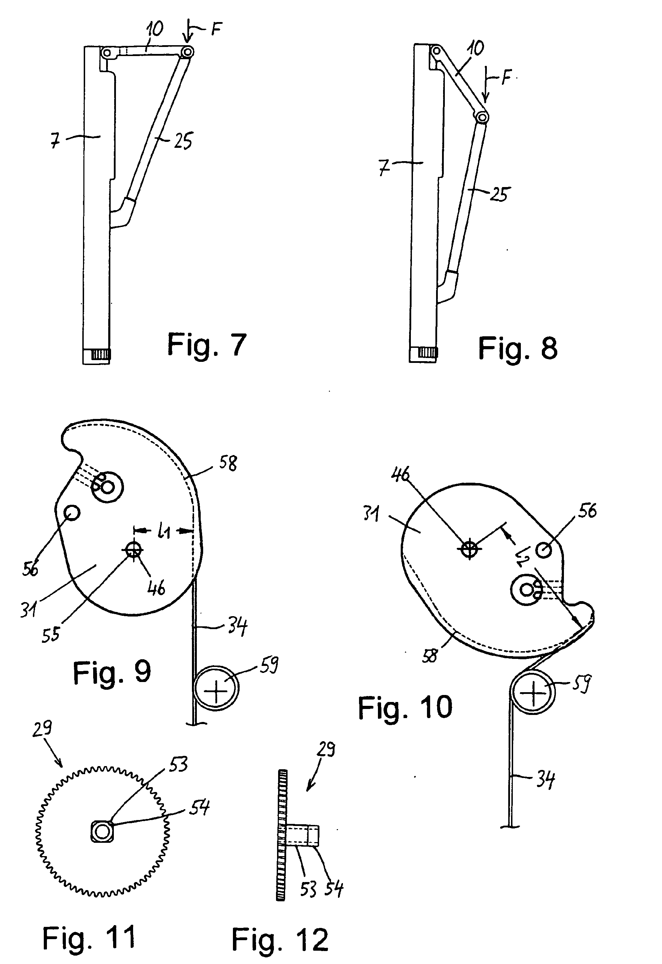

Bei einer Adduktionsbewegung des Arms, würde normalerweise die Federkraft mit zunehmendem Verschiebeweg des Schlittens 36 zunehmen, da sich die Feder 30 zunehmend verformt. Umgekehrt würde bei einer Abduktionsbewegung des Arms die Federunterstützung bei zunehmender Entspannung der Feder 30 abnehmen. Um diese unerwünschten Effekte zu vermeiden, ist die Kurvenscheibe 31 in spezieller Weise geformt, wodurch ein Ausgleichsmechanismus geschaffen wird, welcher der Federkraftveränderung derart entgegenwirkt, dass die zum Bewegen der Oberarmschiene 9 aufzubringende Kraft längs des Verschiebewegs der Stützstange 25 zumindest annähernd gleich bleibt. Hierzu verläuft das Seil 34, wie aus den Figuren 9 und 10 ersichtlich, über einen Umfangsflächenabschnitt 48 der Kurvenscheibe 31, dessen Abstand zur Drehachse 46 der Kurvenscheibe 31 sich längs des Umfangs der Kurvenscheibe 31 ändert. In den Figuren 9 und 10 ist der Umfangsflächenabschnitt 48, mit dem das Seil 34 je nach Drehstellung der Kurvenscheibe 31 mehr oder weniger in Kontakt ist, gestrichelt eingezeichnet, da die Kurvenscheibe 31 in diesem Bereich eine Führungsnut aufweist, in der das Seil 34 vertieft liegt.

Die in Figur 9 gezeigte Drehstellung der Kurvenscheibe 31 entspricht derjenigen, die bei einer maximal angehobenen Stellung der Oberarmschiene 9 bzw. der Gelenkplatte 10 eingenommen wird, wie in Figur 7 dargestellt. Diese Stellung entspricht beispielsweise einer 90°-Stellung relativ zum Gehäuse 7. In dieser Stellung befindet sich die Stützstange 25 in ihrer höchsten Stellung, wobei die Feder 30 maximal entspannt ist. Das Drehmoment, das über die Feder 30 auf die Kurvenscheibe 31 aufgebracht wird, ist somit aufgrund der geringen Federkraft relativ gering.

Wie aus Figur 9 ersichtlich, ist jedoch auch der wirksame Hebelarm l₁ zwischen der Drehachse 46 und demjenigen Bereich des Umfangsflächenabschnitts 48 relativ kurz, über den bei einem Zug am Seil 34 ein gegengerichtetes Drehmoment auf die Kurvenscheibe 31 aufgebracht wird. Dieser kurze Hebelarm l₁ bewirkt somit auch ein relativ geringes Gegendrehmoment auf die Kurvenscheibe 31 durch die auf die Oberarmschiene 9 wirkende Gewichtskraft F (Figur 7).

wird nun, wie aus Figur 8 ersichtlich, der Arm zusammen mit der Gelenkplatte 10 und der Stützstange 25 nach unten bewegt, wird auch der Schlitten 36 nach unten verschoben und die Kurvenscheibe 31 über das Seil 34 im Uhrzeigersinn in die in Figur 10 dargestellte Stellung gedreht. Das Seil 34 bleibt damit mit einem zunehmend kleineren Teil des Umfangsflächenabschnitts 58 in Kontakt, wobei sich der Umfangsflächenabschnitt 58 zunehmend weiter von der Drehachse 46 entfernt. Bei der in Figur 10 gezeigten maximalen Verdrehung der Kurvenscheibe 31 ist der wirksame Hebelarm l₂, über den das Seil 34 ein Gegendrehmoment auf die Kurvenscheibe 31 ausübt, länger als der wirksame Hebelarm l₁ von Figur 9. Bei gegebener Zugkraft am Seil 34 kann somit durch den verlängerten Hebelarm l₂ ein größeres Gegendrehmoment auf die Kurvenscheibe 31 übertragen werden, welches dem durch die zunehmende Verformung der Feder 30 verursachten größeren Drehmoment, das durch die Feder 30 auf die Kurvenscheibe 31 aufgebracht wird, entgegenwirkt. Die zunehmende Federkraft wird somit durch einen länger werdenden wirksamen Hebelarm kompensiert.

Der Kurvenverlauf des Umfangsflächenabschnitts 58 ist so ausgebildet, dass die beschriebene Kompensation der Federkraftveränderung durch Veränderung der wirksamen Hebellängen 11, 12, in jeder Winkelstellung der Oberarmschiene 9 stattfindet. Der Oberarm kann damit, falls gewünscht, über den gesamten Schwenkbereich in Adduktions- und Abduktionsrichtung schwerelos bewegt werden.

Durch Veränderung der Vorspannkraft der Feder 30 durch Drehen des Zahnrads 29 kann die Kraft, mit der der Arm eines Patienten unterstützt wird, stufenlos verändert werden. Mit zunehmender Heilung kann die Federkraft somit auch zunehmend verringert werden, so dass der Patient die zur Abduktion erforderlichen Muskeln zunehmend mehr belasten muss. Umgekehrt kann die Federkraft so erhöht werden, dass der Arm des Patienten durch die Oberarmschiene 9 nach oben gedrückt wird, um das Schultergelenkt zu quengeln und aufzuweiten. Die Einsatzbereiche der erfindungsgemäßen Schultergelenksorthese sind damit sehr vielfältig.

## Patentansprüche

1. Dynamische Schultergelenksorthese, insbesondere Schulterabduktionsorthese, umfassend:
- eine am Oberkörper festlegbare Führungseinrichtung (6) mit einem Gehäuse (7),
- eine an der Führungseinrichtung (6) gelenkig befestigte, zumindest in Adduktions- und Abduktionsrichtung bewegbare Oberarmschiene (9),
- eine Stützstange (25) zum Abstützen der Oberarmschiene (9), wobei die Stützstange (25) ein unteres Ende aufweist, das an der Führungseinrichtung (6) verschiebbar geführt ist,
- eine Feder (30) zum Aufbringen einer Verschiebekraft auf das untere Ende der Stützstange (25),
**dadurch gekennzeichnet, dass** zwischen der Feder (30) und der Stützstange (25) ein Ausgleichsmechanismus vorgesehen ist, welcher einer Federkraftveränderung entgegenwirkt, die beim Schwenken der Oberarmschiene (9) auftritt, so dass die zum Schwenken der Oberarmschiene (9) in Adduktionsrichtung und/oder Abduktionsrichtung aufzubringende Kraft über den gesamten Schwenkbereich zumindest annähernd gleich bleibt.

2. Schultergelenksorthese nach Anspruch 1, **dadurch gekennzeichnet, dass** der Ausgleichsmechanismus eine um eine Drehachse (46) drehbare Kurvenscheibe (31) umfasst, die mit einem mit dem unteren Ende der Stützstange (25) in Wirkverbindung stehenden Kraftübertragungselement bewegungsgekoppelt ist, wobei zwischen Kraftübertragungselement und Drehachse (46) der Kurvenscheibe (31) ein Hebelarm wirkt, dessen Länge l₁, l₂ mit zunehmender Federkraft größer wird.

3. Schultergelenksorthese nach Anspruch 2, **dadurch gekennzeichnet, dass** das Kraftübertragungselement einen Seilzug umfasst, der über einen Umfangsflächenabschnitt (58) der Kurvenscheibe (31) geführt ist, wobei sich der Abstand des Umfangsflächenabschnitts (58) zur Drehachse (46) der Kurvenscheibe (31) längs des Umfangs der Kurvenscheibe (31) ändert.

4. Schultergelenksorthese nach Anspruch 2 oder 3, **dadurch gekennzeichnet, dass** die Feder (30) aus einer Spiralfeder besteht, die an der Hauptebene parallel zu Hauptebene der Kurvenscheibe (31) angeordnet ist.

5. Schultergelenksorthese nach Anspruch 4, **dadurch gekennzeichnet, dass** die Spiralfeder an einem Ende drehfest mit einem Zahnrad (29) gekoppelt ist, das zur Einstellung der Vorspannkraft der Spiralfeder im Gehäuse (7) der Führungseinrichtung (6) drehbar gelagert ist.

6. Schultergelenksorthese nach Anspruch 5, **dadurch gekennzeichnet, dass** das Zahnrad (29) und die Kurvenscheibe (31) parallel zueinander angeordnet und um dieselbe Drehachse (46) drehbar sind.

7. Schultergelenksorthese nach Anspruch 5 oder 6, **dadurch gekennzeichnet, dass** das Zahnrad (29) mittels eines Schneckenrads (48) drehbar ist, das über einen manuell betätigbaren Drehmechanismus drehbar ist.

8. Schultergelenksorthese nach einem der Ansprüche 3 bis 7, **dadurch gekennzeichnet, dass** im Gehäuse (7) der Führungseinrichtung (6) ein Schlitten (36) längsverschiebbar geführt ist, an dem das untere Ende der Stützstange (25) angelenkt und an dem der Seilzug befestigt ist, so dass der Schlitten (36) bei einer Abduktions- oder Adduktionsbewegung der Oberarmschiene (9) sowohl mit der Oberarmschiene (9) als auch mit der federbelasteten Kurvenscheibe (31) bewegungsgekoppelt ist.

9. Schultergelenksorthese nach Anspruch 8, **dadurch gekennzeichnet, dass** der Schwenkbereich der Oberarmschiene (9) durch einen Schlittenwegbegrenzungsmechanismus begrenzt wird, der im oder am Gehäuse (7) angeordnete, in Längsrichtung des Schlittenweges verstellbare Anschläge (39, 40) aufweist.

10. Schultergelenksorthese nach Anspruch 9, **dadurch gekennzeichnet, dass** der Schlittenwegbegrenzungsmechanismus zwei parallel im Gehäuse (7) angeordnete Spindeln (41, 42) umfasst, die jeweils einen Anschlag (39, 40) in der Form einer Spindelmutter tragen.

## Claims

1. Dynamic shoulder joint orthesis, in particular a shoulder abduction orthesis, comprising:
- a guide means (6) which can be fastened on the upper body and comprises a housing (7),
- an upper arm splint (9) which is fastened in an articulated manner to the guide means (6) and can be moved at least in the adduction and abduction directions,
- a support rod (25) for supporting the upper arm splint (9), the support rod (25) comprising a lower end which is guided displaceably on the guide means (6),
- a spring (30) for applying a displacement force on the lower end of the support rod (25).
**characterised in that** a compensation mechanism is provided between the spring (30) and the support rod (25) and counteracts a change in spring force which occurs when the upper arm splint (9) is pivoted, such that the force to be applied to pivot the upper arm splint (9) in the adduction direction and/or in the abduction direction remains at least approximately the same over the entire pivoting range.

2. Shoulder joint orthesis according to claim 1, **characterised in that** the compensation mechanism comprises a cam (31) which is rotatable about an axis of rotation (46) and can be coupled in motion to a force transfer element cooperating with the lower end of the support rod (25), a lever arm acting between the force transfer element and the axis of rotation (46) of the cam (31), the length 1₁, 1₂ of which lever becomes greater with increasing spring force.

3. Shoulder joint orthesis according to claim 2, **characterised in that** the force transfer element comprises a Bowden cable which is guided over a peripheral surface portion (58) of the cam (31), the distance between the peripheral surface portion (58) and the axis of rotation (46) of the cam (31) changing along the periphery of the cam (31).

4. Shoulder joint orthesis according to either claim 2 or claim 3, **characterised in that** the spring (30) consists of a spiral spring which is arranged in terms of the primary plane parallel to the primary plane of the cam (31).

5. Shoulder joint orthesis according to claim 4, **characterised in that** the spiral spring is coupled at one end to a gearwheel (29) in a rotationally engaged manner, which gearwheel is mounted rotatably in the housing (7) of the guide means (6) to adjust the biasing force of the spiral spring.

6. Shoulder joint orthesis according to claim 5, **characterised in that** the gearwheel (29) and the cam (31) are arranged parallel to one another and are rotatable about the same axis of rotation (46).

7. Shoulder joint orthesis according to either claim 5 or claim 6, **characterised in that** the gearwheel (29) is rotatable by means of a worm wheel (48) which is rotatable by a manually actuatable rotation mechanism.

8. Shoulder joint orthesis according to any one of claims 3 to 7, **characterised in that** a sliding carriage (36) is guided in the housing (7) of the guide means (6) in a longitudinally displaceable manner, to which sliding carriage the lower end of the support rod (25) is articulated and to which the Bowden cable is fastened in such a way that the sliding carriage (36) is coupled in motion both with the upper arm splint (9) and with the spring-loaded cam (31) during an abduction or adduction movement of the upper arm splint (9).

9. Shoulder joint orthesis according to claim 8, **characterised in that** the pivoting range of the upper arm splint (9) is defined by a sliding carriage path delimitation mechanism which comprises stops (39, 40) which are arranged in or on the housing (7) and are adjustable in the longitudinal direction of the sliding carriage path.

10. Shoulder joint orthesis according to claim 9, **characterised in that** the sliding carriage path delimitation mechanism comprises two spindles (41, 42) which are arranged in parallel in the housing (7) and which each bear a stop (39, 40) in the form of a spindle nut.

## Revendications

1. Orthèse dynamique pour l'articulation de l'épaule, en particulier orthèse d'abduction d'épaule, comprenant :
- un dispositif de guidage (6) susceptible d'être immobilisé sur le torse, comprenant un boîtier (7),
- une tringle humérale (9) fixée de manière articulée sur le dispositif de guidage (6) et mobile au moins dans la direction d'adduction et la direction d'abduction,
- une tige de soutien (25) pour soutenir la tringle humérale (9), ladite tige de soutien (25) comprenant une extrémité inférieure qui est guidée en déplacement sur le dispositif de guidage (6),
- un ressort (30) pour appliquer une force de déplacement sur l'extrémité inférieure de la tige de soutien (25),
**caractérisée en ce que**, entre le ressort (30) et la tige de soutien (25), il est prévu un mécanisme de compensation qui s'oppose à une modification de la force du ressort, qui se produit lors du pivotement de la tringle humérale (9), de sorte que la force à appliquer pour faire pivoter la tringle humérale (9) dans la direction d'adduction et/ou la direction abduction reste au moins approximativement constante sur la totalité de la plage de pivotement.

2. Orthèse pour l'articulation de l'épaule selon la revendication 1, **caractérisée en ce que** le mécanisme de compensation comprend une plaque à came (31) capable de tourner autour d'un axe de rotation (46), ladite plaque étant couplée en termes de déplacement à un élément de transfert de force qui se trouve en liaison d'action avec l'extrémité inférieure de la tige de soutien (25), et entre l'élément de transfert de force et l'axe de rotation (46) de la plaque à came (31) agit un bras de levier dont la longueur 1₁, 1₂ augmente lorsque la force du ressort augmente.

3. Orthèse pour l'articulation de l'épaule selon la revendication 2, **caractérisée en ce que** l'élément de transfert de force comprend un mécanisme à câble qui est guidé sur un tronçon de surface périphérique (58) de la plaque à came (31), et la distance du tronçon de surface périphérique (58) à l'axe de rotation (46) de la plaque à came (31) varie le long de la périphérie de la plaque à came (31).

4. Orthèse pour l'articulation de l'épaule selon la revendication 2 ou 3, **caractérisée en ce que** le ressort (30) est un ressort spiralé qui est agencé sur le plan principal parallèlement au plan principal de la plaque à came (31).

5. Orthèse pour l'articulation de l'épaule selon la revendication 4, **caractérisée en ce que** le ressort spiralé est couplé à une extrémité solidairement en rotation avec une roue dentée (25), laquelle est montée en rotation dans le boîtier (7) du dispositif de guidage pour régler la force de précontrainte du ressort spiralé.

6. Orthèse pour l'articulation de l'épaule selon la revendication 5, **caractérisée en ce que** la roue dentée (29) et la plaque à came (31) sont agencées parallèlement l'une à l'autre et sont capables de tourner autour du même axe de rotation (46).

7. Orthèse pour l'articulation de l'épaule selon la revendication 5 ou 6, **caractérisée en ce que** la roue dentée (29) est capable de tourner au moyen d'une vis sans fin (48), qui peut être mise en rotation via un mécanisme de rotation à actionnement manuel.

8. Orthèse pour l'articulation de l'épaule selon l'une des revendications 3 à 7, **caractérisée en ce que** dans le boîtier (7) du dispositif de guidage (6) il est prévu un chariot (36) guidé en déplacement longitudinal, auquel est articulée l'extrémité inférieure de la tige de soutien (25) et auquel est fixé le mécanisme à câble, de sorte que le chariot (36) est couplé en termes de déplacement lors d'un mouvement d'abduction ou d'un mouvement d'adduction de la tringle humérale (9) aussi bien avec la tringle humérale (9) qu'avec la plaque à came (31) chargée par un ressort.

9. Orthèse pour l'articulation de l'épaule selon la revendication 8, **caractérisée en ce que** la plage de pivotement de la tringle humérale (9) est limitée par un mécanisme de limitation de course du chariot, qui comprend des butées (39, 40) agencées dans ou sur le boîtier (7), déplaçables en direction longitudinale de la course du chariot.

10. Orthèse pour l'articulation de l'épaule selon la revendication 9, **caractérisée en ce que** le mécanisme de limitation de course du chariot comprend deux broches (41, 42) agencées parallèlement dans le boîtier (7), qui portent respectivement une butée (39, 40) sous la forme d'un écrou de broche.
